# EUROPÄISCHE PATENTSCHRIFT

(11) **EP 1 100 474 B1**
(45) Veröffentlichungstag und Bekanntmachung des Hinweises auf die Patenterteilung: **17.07.2002**
(21) Anmeldenummer: 99944325.2
(22) Anmeldetag: 03.08.1999
(51) Int. Cl.: A61K 9/48, A61M 15/00

(54) **ZWEITEILIGE KAPSEL ZUR AUFNAHME VON PHARMAZEUTISCHEN ZUBEREITUNGEN FÜR PULVERINHALATOREN**
TWO-PIECE CAPSULE FOR RECEIVING PHARMACEUTICAL PREPARATIONS FOR POWDER INHALERS
CAPSULE EN DEUX PARTIES POUR PREPARATIONS PHARMACEUTIQUES DESTINEES AUX INHALATEURS DE MEDICAMENTS EN POUDRE

(30) Priorität: 05.08.1998 DE 19835346
(43) Veröffentlichungstag der Anmeldung: 23.05.2001
(73) Patentinhaber: Boehringer Ingelheim Pharma KG, 55218 Ingelheim am Rhein (DE)
(72) Erfinder: HOCHRAINER, Dieter, D-55411 Bingen am Rhein (DE); ECKERT, Josef, D-97638 Mellrichstadt (DE)
(86) Internationale Anmeldenummer: EP9905614
(87) Internationale Veröffentlichungsnummer: WO0007572

(56) Entgegenhaltungen:
- EP-A- 0 143 524
- EP-A- 0 147 755
- WO-A-82/01470
- FR-A- 2 380 032
- GB-A- 2 064 336
- US-A- 4 192 309

## Beschreibung

Die Erfindung betrifft neue zweiteilige Kapseln zur Aufnahme von pharmazeutischen Zubereitungen zur Verwendung in Pulverinhalatoren.

### Stand der Technik

Kapseln mit pharmazeutischen Zubereitungen werden vielfältig in der Therapie und Diagnose von Krankheiten eingesetzt. Die Kapseln können oral verabreicht werden oder kommen in bestimmten medizinischen Vorrichtungen wie Pulverinhalatoren zum Einsatz. In der Regel bestehen die Kapseln aus zwei Teilen, einem Kapselkörper (Körper) und einer Kapselkappe (Kappe), die teleskopartig ineinander geschoben werden. Aber auch mehrteilige Kapseln sind bekannt. Die Kapseln bestehen meistens aus Gelatine, insbesondere Hartgelatine. Für einige spezielle Anwendungen bestehen die Kapseln zuweilen auch aus für den Menschen gut verdaulichen, wasserlöslichen Kunststoffen, um z.B. bei oraler Verabreichung den Wirkstoff in bestimmten Kompartimenten des Magen-Darm-Trakts freizusetzen. Im folgenden sind Beispiele für verschiedene Kapselmaterialien aufgeführt.

EP 0143524 offenbart eine zweiteilige Kapsel aus für den Menschen gut verdaulichem Material, bevorzugt Gelatine.

EP 0460921 beschreibt Kapseln aus Chitosan und Stärke, Getreidepulver, Oligosacchariden, Methacrylsäure-Methylacrylat, Methacrylsäure-Ethylacrylat, Hydroxypropylmethyl-Celluloseacetat, -succinat oder -phtaleat. Das Kapselmaterial zeichnet sich dadurch aus, daß der Inhalt erst im Dickdarm freigesetzt wird.

GB 938828 offenbart Kapseln für radioaktive Substanzen im therapeutischen oder diagnostischen Einsatz. Die Kapseln bestehen aus wasserlöslicher Gelatine, Methylcellulose, Polyvinylalkohol oder wasserlöslichen nicht-toxischen Thermoplasten.

EP 0 147 755 offenbart einen Inhalator für die Inhalation pulverförmiger Arzneimittel aus Kapseln. Die Zusammensetzung der benutzten Kapseln wurde nicht freigegeben.

Die verwendeten Materialien sind häufig gegenüber Luftfeuchtigkeit nicht sehr beständig, weshalb die pharmazeutische Qualität der Inhaltsstoffe nicht für alle Klimazonen gewährleistet werden kann. Insbesondere in der Klimazone 4 (30°C/70% relative Luftfeuchte) können die herkömmlichen Kapseln nicht verwendet werden.

Zweiteiligen Kapseln, die speziell an die Verwendung in Pulverinhalatoren angepaßt sind, ohne notwendigerweise den Bedingungen für die orale Verabreichung zu unterliegen, sind aus dem Stand der Technik bisher nicht bekannt. Die Kapseln für Pulverinhalatoren bestehen aus den gleichen Materialien, wie sie auch für die orale Verabreichung verwendet werden, meistens aus Hartgelatine. Diese Materialien sind jedoch nicht speziell im Hinblick auf die Verwendung in Pulverinhalatoren optimiert.

Es ist eine der Aufgaben der vorliegenden Erfindung, Kapseln zu schaffen, die besser an die speziellen Bedingungen in Pulverinhalatoren angepaßt werden können.

Die Kapseln, welche bisher in Pulverinhalatoren eingesetzt werden, besitzen aufgrund ihrer Beschaffenheit verschiedene Nachteile. So können die für die Kapseln verwendeten Materialien ihre Eigenschaften in Abhängigkeit von der sie umgebenden Luftfeuchtigkeit ändern und/oder sind nicht immer ausreichend förmstabil. Als Folge davon kann eine solche Kapsel z.B. in der Klimazone 4 aufgrund der hohen Luftfeuchtigkeit nicht verwendet werden, weil das Kapselmäterial die Feuchtigkeit so stark aufnimmt, daß die Formstabilität stark beeinträchtigt wird und/oder die Feuchtigkeit ins Innere der Kapsel eindringt. Dies wirkt sich negativ auf die pharmazeutische Qualität des Inhalts der Kapsel aus. Besagte Materialien besitzen auch in anderen verschiedenen Stadien des Lebens der Kapsel von der Herstellung bis zur Verwendung diverse Nachteile und beeinflussen die Verwendungsfähigkeit der Kapsel als Träger von pharmazeutischen Zubereitungen, die Art der Verabreichung der Inhaltsstoffe, die Haltbarkeit der Inhaltsstoffe und/oder die Verwendungsfähigkeit der Kapsel in bestimmten Ländern. Ein weiterer Nachteil der herkömmlichen Kapselmaterialien besteht z.B. darin, daß sie dazu neigen, pulverförmige Stoffe an sich zu binden, insbesondere wenn sie mit einem Formtrennmittel beschichtet sind, das oft für die Herstellung der Kapsel notwendig ist. In Kapseln für Inhalationszwecke führt dies dazu, daß ein exaktes Dosieren der lungengängigen Feinfraktion erschwert werden kann.

Eine weitere Aufgabe der vorliegenden Erfindung besteht darin, Kapseln für Pulverinhalatoren zu schaffen, die die oben genannten Probleme herkömmlicher Kapseln nicht aufweisen.

### Beschreibung der Erfindung

Die vorliegende Erfindung betrifft eine Kapsel zur Aufnahme von pharmazeutischen Zubereitungen für Pulverinhalatoren mit erhöhter Arzneimittelsicherheit und Kapseln für pharmazeutische Zubereitungen für Pulverinhalatoren mit verbesserter Anpassung an die Verwendung in Pulverinhalatoren. Die Kapseln bestehen aus nicht-wasserlöslichen, hydrophoben Kunststoffen, die die pharmazeutische Qualität der Inhaltsstoffe selbst nicht wesentlich beeinflussen, aber die Einsatzfähigkeit der gefüllten Kapsel in Hinblick auf ihre Funktion, die Dauer der Verwendung und/oder die Klimazone verbessern und in verschieden Stufen von der Herstellung bis zur Verwendung von Vorteil sind.

Die erfindungsgemäße Kapsel besteht aus zwei Teilen, einem Kapselkörper (Körper) und einer Kapselkappe (Kappe), die so miteinander verbunden werden können, daß ein stabiler, abgeschlossener Hohlraum von definiertem Volumen gebildet wird, der die pharmazeutische Formulierung beinhaltet. Die Dimension der Kapsel ist derart, daß sie in gängigen mit Kapseln bestückten Pulverinhalatoren eingesetzt werden können, wie sie z.B. in den Patentschriften DE 33 45 722 (Inhalator Ingelheim M), EP 0 591 136 (Inhalator Ingelheim) oder in der deutsche Offenlegungsschrift DE 43 18 455 ("HandiHaler®") beschrieben sind.

### Detaillierte Beschreibung der Erfindung

In einer Ausführungsform ist der Kunststoff der Kapsel für den Menschen nicht verdaubar, so daß bei oraler Einnahme der Wirkstoff nicht freigesetzt wird. Das hat den Vorteil, daß ein versehentliches Schlucken der Kapsel zu keiner nachhaltigen Beeinträchtigung der Gesundheit führen kann. Insbesondere gilt dies für Kleinkinder oder ältere Menschen.

Bevorzugt werden Kunststoffe verwendet, die spritz- oder blasgußtechnisch verarbeitet werden können und/oder Kunststoffe für deren Verarbeitung zur Kapselkappe oder zum Kapselkörper kein Formtrennmittel notwendig ist, das ein Anhaften der Inhaltsstoffe an die Kapselwand bewirken kann. Das hat den Vorteil, daß das Innere der Kappe oder des Körpers nicht vom Formtrennmittel gereinigt werden muß, um z.B. den amtlichen Bestimmungen (z.B. nach DAB (Deutsches Arzneibuch)) genüge zu tun, die die Verwendung von Formtrennmitteln für Primärpackmittel einschränken.

In einer bevorzugten Ausführungsform besitzt der Kunststoff keine ausgeprägte Adhäsion für pharmazeutisch-chemische Stoffe, insbesondere für Partikel mit lungengängiger Größe, so daß bei Verwendung der Kapsel in einem der oben beschriebenen Inhalatoren der gesamte Inhalt der Kapsel freigesetzt werden kann. Das hat den Vorteil, daß eine exaktere Dosierung, insbesondere des lungengängigen Feinanteils möglich ist.

In einer weiteren Ausführungsform besteht die Kapsel aus einem Kunststoff mit einer Shorehärte D von 65 bis 73. Ein Kunststoff dieser Härte zerspringt nicht, wenn er durchstochen oder aufgeschnitten wird, ist aber gleichzeitig starr genug, daß sich das entstandene Loch nicht von selbst verschließt. Der Vorteil eines solchen Materials ist, daß während des Öffnens, Aufstechens oder Aufschneidens der Kapsel im Pulverinhalator keine Teile aus der Kapsel abgesprengt werden, die dann während des Inhalierens eingeatmet werden könnten.

In einer Ausführungsform ist die Kunststoffkapsel so stabil, daß sie entlang der Längsache oder der Querachse einer Kraft bis zu 15 N standhält. Der Vorteil besteht darin, daß die Kapsel besser an die Beanspruchungen angepaßt ist, die bei der Herstellung, dem Füllen, Verpacken, Transportieren u.ä. auf die Kapsel einwirken.

In einer weiteren Ausführungsform besitzt die Wandung der Kapsel eine Durchlässigkeit für Wasserdampf von weniger als 1,3 x 10⁻¹⁴ kg/(m²s Pa), bevorzugt von 1,5 x 10⁻¹⁶ bis 2 x 10⁻¹⁶ kg/(m² s Pa). Der Vorteil dieser Eigenschaft besteht darin, daß die Inhaltsstoffe der Kapsel auch in geographischen Zonen mit hoher Luftfeuchtigkeit vor Wasser geschützt sind.

In bevorzugten Ausführungen ist der Kunststoff Polyethylen, insbesondere high-density Polyethylen, Polycarbonat, Polyester, Polypropylen oder Polyethylenterephthalat.

In einer bevorzugten Ausführungsform haben die Kappe und der Körper die Form eines Zylinders mit rundem Querschnitt und konvexer, nahezu halbkugelförmiger geschlossener Unterseite und bestehen beide aus high-density Polyethylen.

Die erfindungsgemäße Kapsel kann in allen Arten von Pulverinhalatoren verwendet werden, bei denen die zu inhalierende Zubereitung durch eine Kapsel eingebracht wird.

In einer bevorzugten Ausführungsform sind Kappe und Körper der Kapsel von gegenseitig ähnlicher, zylinderartiger Form, bestehend aus einem in sich geschlossenem Mantel mit jeweils einer geschlossenen und einer offenen Seite. Dabei sind Form und Größe der Kappe und der Kapsel dergestalt, daß der Körper mit seinem offenen Ende teleskopartig so in das offene Ende der Kappe hinein geschoben werden kann, daß die Kappe fest mit dem Körper verbunden ist.

In einer speziellen Ausführungsform sind Kappe und Körper mit Verschlußeinrichtungen versehen, die beim vorläufigen und/oder endgültigen Verschließen der Kapsel von Vorteil sind.
In einer solchen Ausführungsform befinden sich auf dem Innenmantel der Kappe punktförmige Erhebungen und auf dem Außenmantel des Körper etwas größere punktförmige Vertiefungen, die so angeordnet sind, daß beim Verschließen der Kapsel die Erhebungen in die Vertiefungen einrasten. Alternativ können die Erhebungen auf dem Außenmantel des Körpers und die Vertiefungen auf dem Innenmantel der Kappe ausgebildet sein. Bevorzugt sind Anordnungen, bei denen die Erhebungen oder Vertiefungen jeweils ringförmig oder spiralförmig um den Mantel angeordnet sind. Anstelle der punktförmigen Gestaltung der Erhebungen und Vertiefungen können diese auch durchgehend den Mantel der Kappe bzw. des Körpers ringförmig umlaufen.
In einer Ausführungsform sind auf dem Innenmantel der Kappe und dem Außenmantel des Körper ein oder mehrere ringförmig umlaufende Erhebungen derart ausgebildet, daß sich im geschlossenen Zustand der Kapsel eine Erhebungen der Kappe jeweils neben einer Erhebung des Körpers befindet.
In den Ausführungsformen mit besagten ringförmigen Vertiefungen und/oder Erhebungen können diese durchgängig oder unterbrochen sein.
In einer weiteren Ausführungsform sind auf der Außenseite des Körpers nahe dem offenen Ende Erhebungen und in der Kappe nahe dem offenen Ende Löcher so ausgebildet, daß die Erhebungen des Körpers im geschlossenen Zustand der Kapsel in die Löcher der Kappe einrasten. Die Erhebungen können dabei derart sein, daß die Kappe jederzeit ohne Beschädigung der Kapsel geöffnet werden kann oder aber daß die Kapsel nach einmaligem Verschließen nicht mehr zerstörungsfrei geöffnet werden kann.

In einer weiteren Ausführungsform ist auf der Außenseite des Körpers ein Wulst ausgebildet, der senkrecht zu der Verbindungsachse zwischen Kappe und Körper ringsum den Körper läuft. Der Wulst dient als Stopper für die Kapsel, wenn diese über den Körper gesteckt wird, um ein Durchstoßen der Kappe mit dem Körper zu verhindern. Der Bereich zwischen offenem Ende des Körpers und dem Wulst entspricht dem Bereich des Körpers über den die Kappe geschoben werden kann. Der Wulst ist so auf dem Körper lokalisiert, daß die Kappe weit genug über den Körper geschoben werden kann, um einen festen Verschluß zwischen Kappe und Körper zu bewirken. D.h. der Wulst befindet sich z.B. nicht unmittelbar an der offenen Seite des Körpers. Die Seite des Wulstes, die zum offenen Ende des Körpers zeigt, steht als senkrechte Kante so auf der Außenwand des Körpers, daß die Kappe beim Verschließen nicht über den Wulst hinweg geschoben werden kann. Die Seite des Wulstes, die zum geschlossenen Ende des Körpers weist, kann in Form einer nahezu rechtwinkeligen Kante ausgebildet sein oder sich zum geschlossenen Ende des Körpers hin verflachen. Die Ausbildung einer nahezu rechtwinkeligen Kante kann bei einer losen Einpassung der Kapsel in einen Kapselhalter von Vorteil sein, die Variante mit sich verflachendem Wulst bei einer festen Einpassung. Der Wulst kann durchgängig oder unterbrochen sein.

In einer bevorzugten Ausführungsform verflacht sich der Wulst kontinuierlich zum geschlossenen Ende des Körpers und steht mit seiner zum offenen Ende des Körpers orientierten Seite senkrecht auf dem Kapselkörper auf. Dabei ist die Höhe der so gebildeten Kante derart, daß die Kante im geschlossenen Zustand der Kapsel nicht über die Kapselkappe hinaus ragt, so daß der Übergang von Kapselkappe zu Kapselkörper plan ist.

Die Stärke der Wände der Kappe und des Körpers können über den Gesamtbereich variieren. So ist die Wandstärke in der Regel in den abgerundeten Bereichen der Kappe oder des Körpers oder an der Stelle des Körpers, an der der Wulst ausgebildet ist größer als in den Bereichen, in denen die Wände geradlinig verlaufen. In einer Ausführungsform haben die Wände der Kappe und des Körpers eine Dicke von 0,1 mm bis 0,5 mm.

In einer möglichen Ausführungsform sind an der Außenseite der Kapsel Noppen ausgebildet, in einer anderen drei oder mehr Rippen, die parallel zur Längsachse der Kapsel verlaufen. Der Vorteil dieser Einrichtungen besteht darin, daß die Kapsel aus einer Kapselhalterung, wie sie z.B. in den oben genannten Pulverinhalatoren verwendet werden, so herausgenommen werden kann, daß sie nicht beschädigt wird oder aufgeht. Die Rippen oder Noppen können über die gesamte Außenseite der Kapsel hinweg verlaufen oder nur einen Teil davon bedecken. Alternativ können sie nur an der Kappe ausgebildet sein oder nur in dem Bereich des Körpers, der im geschlossenen Zustand nach außen sichtbar ist. Die Rippen verlaufen parallel zur Längsache der Kapsel und bewirken, daß die Kapsel senkrecht in besagter Kapselhalterung fixiert ist. Im Fall eines kreisförmigen Querschnitts der Kapsel sind die Rippen bevorzugt so angeordnet, daß der Querschnitt der Kapsel keine Rotationssymmetrie um die Mittelachse aufweist. In einer solchen Ausführungsform können die Rippen nur in dem Bereich des Körpers ausgebildet sein, der im geschlossenen Zustand der Kapsel sichtbar ist. Eine solche Ausführungsform verhindert das Festklemmen der Kapsel in einem Kapselhalter.
In einer Ausführungsform ohne Wulst, aber mit Rippen an dem Teil des Körpers, der im geschlossenen Zustand der Kappe sichtbar ist, sind die Rippen so ausgebildet, daß die zum offenen Ende des Körpers orientierten Enden der Rippen die Aufgabe des Wulstes erfüllen, nämlich als Stopper für die Kappe zu dienen beim Zusammenfügen der Kappe mit dem Körper.

In einer weiteren Ausführung beschreiben die Mäntel der Kappe und der Körper einen hohlen Zylinders mit rundem, ovalem, drei-, vier-, sechs-, acht- oder mehreckigem Querschnitt, wobei die jeweilige Oberseite offen und die Unterseite geschlossen ist. Die geschlossene Unterseite kann flach oder konvex sein. Die eckigen Ausführungsformen haben z.B. den Vorteil, daß sie platzsparender gelagert werden können als die runden.

In einer Ausführungsform ist die Elongation der Kapsel (Abstand vom geschlossenen Ende des Körpers zum geschlossenen Ende der Kappe in Relation zum Durchmesser bei geschlossener Kapsel) größer 1, in einer Ausführungsform ist die Elongation gleich 1und in wieder einer anderen Ausführungsform ist die Elongation kleiner 1. Letzteres hat den Vorteil, daß der Körper eine größere Öffnung zum Füllen aufweist.
Bei einer der Ausführungsformen mit einer Elongation gleich 1 sind Kappe und Körper dergestalt, daß die geschlossene Kapsel die Form einer Kugel hat, was für ein automatisches Beladen eines Pulverinhalators mit der Kapsel aus einem Reservoir von Vorteil sein kann.

Um im geschlossenen Zustand der gefüllten Kapsel eine bessere Abdichtung zwischen Kappe und Körper zu erreichen, kann die Nahtstelle zwischen Kappe und Körper zum Verschließen verschweißt, verklebt oder banderoliert werden, wodurch die Wasserdampfpermeabilität auf bis zu einem Zehntel abnimmt. Alternativ kann die gesamte Kappe mit einem durchgehenden Schutzfilm überzogen werden.

In einer weiteren bevorzugten Ausführungsform kann der Spalt mit einem Füllstoff verschlossen werden. Als Füllstoff für ein solches Verfüllen des Spalts eigenen sich pharmazeutisch zulässige Füllmaterialien, wie beispielsweise Eudragit. Ein solcher Füllstoff kann als Lösung oder Suspension in einem geeigneten, bevorzugt leicht flüchtigen, Lösungsmittel in den Spalt eingetragen werden.
Als Lösungsmittel eignen sich Fluorchlorkohlenwasserstoffe wie beispielsweise Methylenchlorid oder Chloroform, Fluorkohlenwassserstoffe, Alkohole, wie Methanol, Ethanol, Propanol, Isopropanol, Alkane, wie Propan, Hexan, Hepatan, Ketone, wie Aceton, Ester, wie Essigsäureethylester, Ether, wie Dimehtylether oder Diethlether oder andere aus dem Stand der Technik bekannten zum Lösen oder Suspendieren geeigneten Flüssigkeiten, insbesondere leicht flüchtige und solche, die das Kapselmaterial nicht angreifen, mit Arzneimittel nicht chemisch interagieren oder es in seiner Bioverfügbarkeit verändern.
Die Lösung oder Suspension mit dem Füllmaterial muß derart konzentriert und beschaffen sein, daß mit der Lösung oder Suspension genug Füllmaterial in den Spalt eingetragen werden kann, so daß dieser, nachdem sich das Lösungsmittel verflüchtigt hat, durch das zurückbleibenden Füllmaterial dicht verschlossen wird und gleichzeitig darf die Lösung oder Suspenion nicht so konzentriert oder beschaffen sein, daß sie zu zähflüssig ist, um in den Spalt einzudringen, bzw. durch Kapillarkräfte in diesen hingezogen zu werden.

Als bevorzugt wird eine Lösung aus Eudragit und Aceton zum Verschließen des Spalts eingesetzt.

Aus der Beschreibung wird ersichtlich, daß die erfindungsgemäße Kapsel geeignet ist, jedwegliche pulverförmige und zur Inhalation geeignete pharmazeutische Formulierung aufzunehmen. In einer besonderen Verwendungsform enthält die Kapsel Cromoglicinsäure, Reproterol, Beclomethason, Terbutalin, Salbutamol, Salmeterol, Ketotifen, Orciprenalin Fluticason, Insulin, Ipratropium, Dexamethason, Bambuterol, Tiotropium, Budesonid, Fenoterol, Clenbuterol Prednisolon, Prednison, Prednyliden, Methylprednisolon, Formoterol, Nedocromil, deren Salze oder Gemische oder ein anderes für Inhalationszwecke geeignetes Kortisonpräparat oder Atropinderivat.
In einer bevorzugten Verwendungsform enthält die Kapsel Ipratropiumbromid oder Tiotropiumbromid.

### Beschreibung der Abbildungen

Die Abbildungen zeigen exemplarisch verschiedenen Ausführungsformen der erfindungsgemäßen Kapsel, dienen jedoch nur zur Illustration ohne den Umfang der Erfindung einzuschränken.
Fig. 1 zeigt die einfachste Ausführungsform der erfindungsgemäßen Kapsel im lateralen Querschnitt
Fig. 2a und 2b zeigen je eine unterschiedliche Ausführungsform der Kapsel mit sich verjüngendem Wulst am Körper im lateralen Querschnitt
Fig. 3 zeigt eine Ausführungsform der Kapsel mit kantenförmigem Wulst am Körper im lateralen Querschnitt
Fig. 4 zeigt eine Ausführungsform der Kapsel mit sich verjüngendem Wulst am Körper und ringförmiger Vertiefung an Körper und Kappe im lateralen Querschnitt
Fig. 5 zeigt eine Ausführungsform der Kapsel mit sich verjüngendem Wulst am Körper und ringförmiger Vertiefung an Körper und Kappe in Frontalansicht
Fig. 6 zeigt eine Ausführungsform der Kapsel mit sich verjüngendem Wulst am Körper und punktförmigen Vertiefungen bzw. Erhebungen an Körper und Kappe in Frontalansicht
Fig. 7 zeigt eine Ausführungsform der Kapsel mit sich verjüngendem Wulst am Körper und punktförmigen Erhebungen am Körper und punktförmigen Löchern in der Kappe in Frontalansicht
Fig. 8 zeigt eine Ausführungsform der Kapsel mit Rippen am Körper in Frontalansicht
Fig. 9 zeigt die Kapsel der Fig. 7 im horizontalen Querschnitt
Fig 10a, 10b und 10c zeigen Ausführungsformen der Kapsel mit je verschiedenem Querschnitt
Ein Ausführungsbeispiel mit einer kugelförmigen Kapsel ist nicht dargestellt.

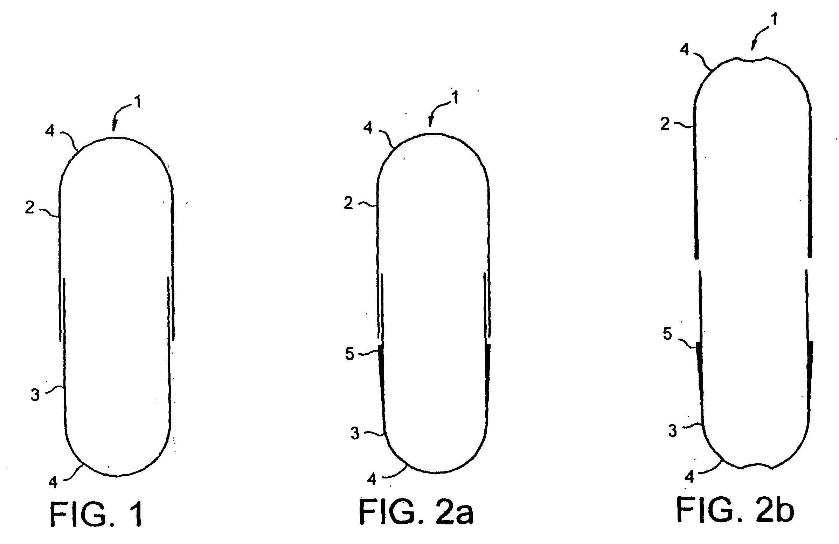

In Figur 1 ist die einfachste Ausführungsform der erfindungsgemäßen Kapsel **1** im Querschnitt gezeigt. Die Kapsel **1** besteht aus der Kappe **2** und dem Körper **3**, die teleskopartig ineinander gesteckt sind. Kappe **2** und Körper **3** sind von gleicher Gestalt und haben je eine konvexe Unterseite **4.**
In Figur 2a ist im Querschnitt eine Ausführungsform gezeigt, bei der am Körper **3** der Kapsel **1** ein Wulst **5** ausgebildet ist, der sich zum geschlossenen Ende des Körpers hin verjüngt. Mit der zum offenen Ende des Körpers hin orientierten Seite steht der Wulst **5** nahezu senkrecht auf dem Körper. Die so ausgebildete Kante begrenzt den Bereich des Körpers über den die Kappe **2** teleskopartig geschoben werden kann.

Eine andere Ausführungsform ist in Figur 2b abgebildet. Der Querschnitt zeigt, daß sich diese Ausführungsform von der in Figur 2a dargestellten dadurch unterscheidet, daß die Wandstärke der Kappe **2** bzw. des Körpers **3** nicht über den gesamten Bereich gleich stark ausgebildet ist, sondern über einzelne Teilbereiche variiert. Zusätzlich weisen die konvexen Unterseiten **4** der Kappe bzw. des Körpers je eine konkave Einbuchtung am Scheitelpunkt auf.

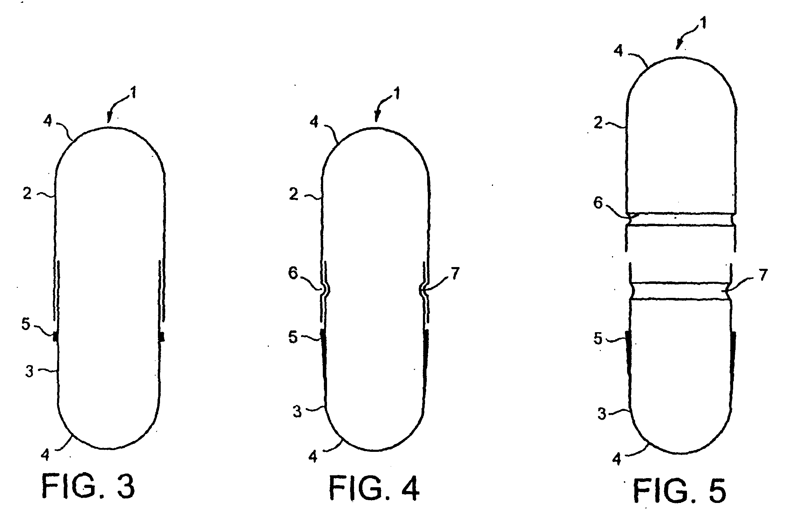

In Figur 3 ist eine Ausführungsform dargestellt, bei der der Wulst **5** sowohl zur Oberseite des Körpers als auch zu seiner Unterseite nahezu rechtwinkelig auf dem Körper aufsitzt.
Die Ausführungsform der Figur 4 stellt eine Weiterentwicklung der Ausführungsform der Figur 2a dar, bei der eine ringförmige Vertiefung **6** bzw. **7** in Kappe **2** bzw. Körper **3** zum besseren Verschluß der Kapsel **1** ausgebildet ist.
In Figur 5 ist eine Frontalansicht der in Figur 4 als Querschnitt gezeigten Ausführungsform abgebildet.

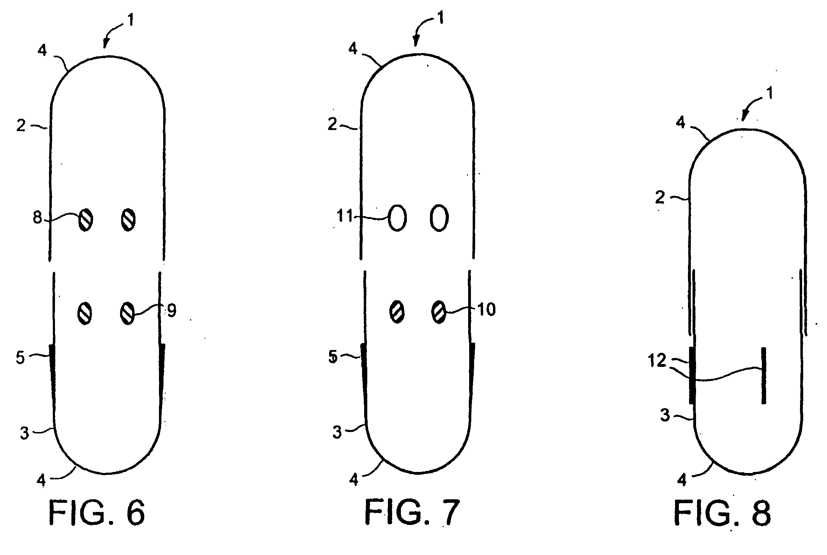

Figur 6 zeigt eine weitere Variante der Erfindung mit punktuellen Vertiefungen **8** und **9** als Frontalansicht.
In Figur 7 ist eine Variante der Kapsel **1** dargestellt, bei der am Körper **3** nahe dem offenen Ende Erhebungen **10** und in der Kappe **2** nahe dem offenen Ende Löcher **11** so ausgebildet sind, daß die Erhebungen **10** beim Verschließen der Kapsel in die Löcher **11** einrasten.
Die Figur 8 stellt eine Ausführungsform der Kapsel **1** von außen dar, bei der auf dem Körper **3** die Rippen **12** ausgebildet sind.

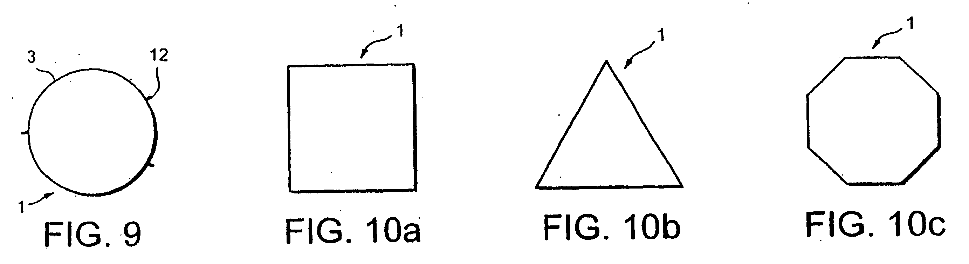

Figur 9 zeigt den Körper **3** der Ausführungsform der Figur 7 im Querschnitt. Der Querschnitt zeigt, daß die drei Rippen **12** nicht rotationssymmetrisch um die Mittelachse des Körpers angeordnet sind. In Figur 10a ist eine Kapsel **1** mit viereckigem Querschnitt dargestellt, in Figur 10b eine mit dreieckigem und in Figur 10c eine mit achteckigem Querschnitt.

## Patentansprüche

1. Kapsel für pharmazeutische Zubereitungen für Pulverinhalatoren bestehend aus einem Kapselkörper und einer Kapselkappe, die beide aus dem gleichen Material bestehen und die so miteinander verbunden werden können, daß ein stabiler, abgeschlossener Hohlraum von definiertem Volumen gebildet wird, **dadurch gekennzeichnet, daß** das Kapselmaterial ein nicht-wasserlöslicher, hydrophober Kunststoff ist.

2. Kapsel nach Anspruch 1, **dadurch gekennzeichnet, daß** die Wände der Kappe und des Körpers 0,1 mm bis 0,5 mm stark sind.

3. Kapsel nach Anspruch 1 oder 2, **dadurch gekennzeichnet, daß** die Kapsel einer entlang ihrer Längsachse und ihrer Querachse einwirkenden Kraft bis zu 15 N standhält.

4. Kapsel nach Anspruch 1, 2 oder 3, **dadurch gekennzeichnet, daß** die Shorehärte D des Kunststoffs in einem Bereich von 65 bis 73 liegt.

5. Kapsel nach Anspruch 1, 2, 3 oder 4, **dadurch gekennzeichnet, daß** die Wandung der Kapsel eine Durchlässigkeit für Wasserdampf von weniger als 1,3 x 10⁻¹⁴ kg/(m² s Pa) besitzt.

6. Kapsel nach einem der vorhergehenden Ansprüche, **dadurch gekennzeichnet, daß** die Wandung der Kapsel eine Durchlässigkeit für Wasserdampf von 1,5 x 10⁻¹⁶ bis zu 2 x 10⁻¹⁶ kg/(m² s Pa) besitzt.

7. Kapsel nach Anspruch 1, 2, 3, 4 oder 5, **dadurch gekennzeichnet, daß** der Kunststoff Polyethylen, Polycarbonat, Polyester, Polypropylen oder Polyethylenterephthalat ist.

8. Kapsel nach Anspruch 7, **dadurch gekennzeichnet, daß** der Kunststoff Polyethylen ist.

9. Kapsel nach einem der vorhergehenden Ansprüche 1, 2, 3, 4, 5, 6, 7 oder 8, **dadurch gekennzeichnet, daß** sich auf dem Innenmantel der Kappe eine oder mehrere Erhebungen oder Vertiefungen und auf dem Außenmantel des Körpers eine oder mehrere Vertiefungen oder Erhebungen befinden, die so angeordnet sind, daß die Erhebungen beim Verschließen der Kapsel in die Vertiefungen einrasten.

10. Kapsel nach einem der vorhergehenden Ansprüche, **dadurch gekennzeichnet, daß** sich auf der Außenseite des Körpers senkrecht zu der Verbindungsachse Kappe und Körper ein ringsum verlaufender Wulst befindet, der mit seiner zum offenen Ende des Körpers hin orientierten Seite nahezu rechtwinkelig auf der Außenwand des Körpers steht.

11. Kapsel nach einem der vorhergehenden Ansprüche, **dadurch gekennzeichnet, daß** der Kapselkörper und die Kapselkappe jeweils aus high-density Polyethylen sind und jeweils die Form eines Zylinders mit rundem Querschnitt und einem konvexen geschlossenen Enden haben, so daß die Elongation der Kapsel (Abstand vom geschlossenen Ende des Körpers zum geschlossenen Ende der Kappe in Relation zum Durchmesser bei geschlossener Kapsel) größer 1 ist.

12. Kapsel nach einem der vorangegangenen Ansprüche 1 bis 11, **dadurch gekennzeichnet, daß** die Nahtstelle zwischen Körper und Kappe, bzw. der Spalt durch Verschweißen, Verkleben, Banderolieren, Überziehen der Kappe mit einem Schutzfilm verschlossen ist.

13. Kapsel nach einem der vorangegangenen Ansprüche 1 bis 12, **dadurch gekennzeichnet, daß** die Nahtstelle zwischen Körper und Kappe, bzw. der Spalt durch Verfüllen mit einem pharmazeutisch zulässigen Füllstoff verschlossen ist.

14. Kapsel nach Anspruch 13, **dadurch gekennzeichnet, daß** der Füllstoff Eudragit ist.

15. Verwendung der Kapsel nach einem der vorangegangenen Ansprüche für eine pharmazeutische Zubereitung, die Cromoglicinsäure, Reproterol, Beclomethason, Terbutalin, Salbutamol, Salmeterol, Ketotifen, Orciprenalin Fluticason, Ipratropium, Dexamethason, Bambuterol, Tiotropium, Budesonid, Fenoterol, Clenbuterol, Prednisolon, Prednison, Prednyliden, Methylprednisolon, Formoterol oder Nedocromil, Insulin Ipratropiumbromid, Tiotropiumbromid, deren Salze oder Gemische oder ein anderes für Inhalationszwecke geeignetes Kortisonpräparat oder Atropinderivat enthält.

16. Verwendung der Kapsel nach einem der vorhergehenden Ansprüche in Pulverinhalatoren.

## Claims

1. A capsule for pharmaceutical preparations for powder inhalers consisting of a capsule body and a capsule cap, which both consist of the same material and which can be attached to one another so as to form a stable, enclosed hollow space of defined volume, **characterised in that** the capsule material is a water-insoluble, hydrophobic synthetic material.

2. A capsule according to claim 1, **characterised in that** the walls of the cap and the body are 0.1 mm to 0.5 mm thick.

3. A capsule according to claim 1 or 2, **characterised in that** the capsule can withstand a force acting upon its longitudinal axis and its transverse axis of up to 15 N.

4. A capsule according to claim 1, 2 or 3, **characterised in that** the Shore hardness D of the synthetic material is in the range from 65 to 73.

5. A capsule according to claim 1, 2, 3 or 4, **characterised in that** the wall of the capsule has a steam permeability of less than 1.3 x 10⁻¹⁴ kg/(m² s Pa).

6. A capsule according to one of the preceding claims, **characterised in that** the wall of the capsule has a steam permeability of 1.5 x 10⁻¹⁶ to 2 x 10⁻¹⁶ kg/(m² s Pa).

7. A capsule according to claim 1, 2, 3, 4 or 5, **characterised in that** the synthetic material is polyethylene, polycarbonate, polyester, polypropylene or polyethyleneterephthalate.

8. A capsule according to claim 7, **characterised in that** the synthetic material is polyethylene.

9. A capsule according to one of the preceding claims 1, 2, 3, 4, 5, 6, 7 or 8, **characterised in that** one or more elevations or recesses are located on the inner jacket of the cap and one or more recesses or. elevations are located on the outer jacket of the body, these elevations or recesses being arranged so that the elevations engage with the recesses when the capsule is closed.

10. A capsule according to one of the preceding claims, **characterised in that** a bulge runs in an annular shape around the outside of the body perpendicular to the connecting axis of the cap and the body, the side of the bulge which is orientated towards the open end of the body standing practically at right angles to the outer wall of the body.

11. Capsule according to one of the preceding claims, **characterised in that** the capsule body and capsule cap are both made of high density polyethylene and are both in the shape of a cylinder of round cross section with a convex closed end, so that the elongation of the capsule (distance from the closed end of the body to the closed end of the cap in relation to the diameter when the capsule is closed) is greater than 1.

12. Capsule according to one of the preceding claims 1 to 11, **characterised in that** the joint between the body and cap, or the gap, is sealed by welding, adhesive bonding, wrapping or covering the cap with a protective film.

13. Capsule according to one of the preceding claims 1 to 12, **characterised in that** the joint between the body and cap, or the gap, is sealed by filling with a pharmaceutically acceptable filler.

14. Capsule according to claim 13, **characterised in that** the filler is Eudragit.

15. Use of the capsule according to one of the preceding claims for a pharmaceutical preparation which contains cromoglycic acid, reproterol, beclomethasone, terbutaline, salbutamol, salmeterol, ketotifen, orciprenaline, fluticasone, ipratropium, dexamethasone, bambuterol, tiotropium, budesonide, fenoterol, clenbuterol, prednisolone, prednisone, prednylidene, methylprednisolone, formoterol, nedocromil, insulin, ipratropium bromide, tiotropium bromide, the salts or mixtures thereof or another cortisone preparation or atropine derivative suitable for inhalation purposes.

16. Use of the capsule according to one of the preceding claims in powder inhalers.

## Revendications

1. Capsule pour préparations pharmaceutiques pour inhalateurs à poudre consistant en un corps de capsule et une coiffe de capsule, qui consistent l'un et l'autre en le même matériau et qui peuvent être liés l'un à l'autre de telle manière qu'il se forme une cavité fermée stable de volume défini, **caractérisée en ce que** le matériau de la capsule est une matière synthétique hydrophobe non hydrosoluble.

2. Capsule selon la revendication 1 **caractérisée en ce que** les parois de la coiffe et du corps sont épaisses de 0,1 mm à 0,5 mm.

3. Capsule selon la revendication 1 ou 2 **caractérisée en ce que** la capsule résiste à une force agissant suivant son axe longitudinal et son axe transversal pouvant atteindre 15 N.

4. Capsule selon la revendication 1, 2 ou 3 **caractérisée en ce que** la dureté Shore D de la matière synthétique est située dans un domaine de 65 à 73.

5. Capsule selon la revendication 1, 2, 3 ou 4 **caractérisée en ce que** la paroi de la capsule possède une perméabilité pour la vapeur d'eau inférieure à 1,3 x 10⁻¹⁴ kg/(m² s Pa).

6. Capsule selon l'une des revendications précédentes **caractérisée en ce que** la paroi de la capsule possède une perméabilité pour la vapeur d'eau de 1,5 x 10⁻¹⁶ à 2 x 10⁻¹⁶ kg/(m² s Pa).

7. Capsule selon la revendication 1, 2, 3, 4 ou 5 **caractérisée en ce que** la matière synthétique est du polyéthylène, polycarbonate, polyester, polypropylène ou polyéthylènetéréphtalate.

8. Capsule selon la revendication 7 **caractérisée en ce que** la matière synthétique est du polyéthylène.

9. Capsule selon l'une des revendications 1, 2, 3, 4, 5, 6, 7 ou 8 précédentes **caractérisée en ce qu'**il y a sur la surface interne de la coiffe une ou plusieurs protubérances ou un ou plusieurs évidements et sur la surface externe du corps un ou plusieurs évidements ou une ou plusieurs protubérances qui sont disposés de telle manière que les protubérances s'enclenchent dans les évidements lors de la fermeture de la capsule.

10. Capsule selon l'une des revendications précédentes **caractérisée en ce qu'**il y a sur le côté externe du corps, perpendiculairement à l'axe de liaison de la coiffe et du corps, un renflement qui s'étend tout autour, dont le côté orienté vers l'extrémité ouverte du corps est situé sensiblement à angle droit sur la paroi externe du corps.

11. Capsule selon l'une des revendications précédentes **caractérisée en ce que** le corps de la capsule et la coiffe de la capsule consistent chacun en polyéthylène haute densité et ont chacun la forme d'un cylindre à section droite ronde et à extrémité fermée convexe, de sorte que l'élongation de la capsule (distance de l'extrémité fermée du corps à l'extrémité fermée de la coiffe par rapport au diamètre quand la capsule est fermée) est supérieure à 1.

12. Capsule selon l'une des revendications 1 à 11 précédentes **caractérisée en ce que** la jonction, ou l'interstice, entre le corps et la coiffe est fermée par soudage, collage, application d'une bande, recouvrement de la coiffe avec un film protecteur.

13. Capsule selon l'une des revendications 1 à 12 précédentes **caractérisée en ce que** le la jonction, ou l'interstice, entre le corps et la coiffe est fermée par remplissage avec une charge pharmaceutiquement acceptable.

14. Capsule selon la revendication 13 **caractérisée en ce que** la charge est l'Eudragit.

15. Utilisation de la capsule selon l'une des revendications précédentes pour une préparation pharmaceutique qui contient de l'acide cromoglycique, du reprotérol, de la beclométhasone, de la terbutaline, du salbutamol, du salmétérol, du kétotifène, de l'orciprénaline, de la fluticasone, de l'ipratropium, de la dexaméthasone, du bambutérol, du tiotropium, du budésonide, du fénotérol, du clenbutérol, de la prednisolone, de la prednisone, du prednylidène, de la méthylprednisolone, du formotérol ou du nédocromil, de l'insuline, du bromure d'ipratropium, du bromure de tiotropium, leurs sels ou mélanges ou une autre préparation de cortisone ou un autre dérivé d'atropine appropriés à des fins d'inhalation.

16. Utilisation de la capsule selon l'une des revendications précédentes dans des inhalateurs à poudre.
